# FASCICULE DE BREVET EUROPEEN

(11) **EP 1 440 050 B1**
(45) Date de publication et mention de la délivrance du brevet: **31.03.2010**
(21) Numéro de dépôt: 02785556.8
(22) Date de dépôt: 29.10.2002
(51) Int. Cl.: C07C 45/53, C07C 49/403, C07C 29/132, C07B 41/02, C07B 41/06

(54) **PROCEDE DE DECOMPOSITION CATALYTIQUE DES HYDROPEROXYDES ORGANIQUES**
VERFAHREN ZUR KATALYTISCHEN ZERSETZUNG VON ORGANISCHEN HYDROPEROXIDEN
METHOD FOR CATALYTIC DECOMPOSITION OF ORGANIC HYDROPEROXIDES

(30) Priorité: 30.10.2001 FR 0114038
(43) Date de publication de la demande: 28.07.2004
(73) Titulaire: Rhodia Opérations, 93300 Aubervilliers (FR)
(72) Inventeur: BONNET, Didier, F-69004 Lyon (FR); FACHE, Eric, F-69300 Caluire (FR); SEIGNEURIN, Aline, F-78150 Le Chesnay (FR)
(74) Mandataire: Esson, Jean-Pierre
(86) Numéro de dépôt international: PCT/FR2002/003713
(87) Numéro de publication internationale: WO 2003/037839

(56) Documents cités:
- WO-A-01/44153
- DE-A- 19 849 593
- FR-A- 2 744 719
- US-A- 2 851 496
- C. B. HANSEN: "Anchored manganese and ruthenium porphyrins as catalysts in the decomposition of cyclohexyl hydroperoxide" JOURNAL OF MOLECULAR CATALYSIS, vol. 79, no. 1-3, 1993, pages 153-163, XP008005276

## Description

La présente invention concerne un procédé de décomposition des hydroperoxydes organiques, en présence d'un catalyseur.

Les hydroperoxydes organiques sont des intermédiaires importants dans la préparation d'alcools, de cétones et d'acides, servant souvent eux-mêmes de matières premières en synthèse organique.

Parmi ces hydroperoxydes organiques, l'hydroperoxyde de cyclohexyle est préparé par oxydation du cyclohexane. Par décomposition catalytique, il conduit à la cyclohexanone et au cyclohexanol. Ces derniers composés peuvent être transformés en acide adipique par oxydation. L'acide adipique est un intermédiaire chimique important utilisé dans la fabrication de nombreux polymères comme les polyamides, polyuréthanes, par exemple. Ce composé peut avoir de nombreuses autres applications.

La décomposition des hydroperoxydes organiques et notamment de l'hydroperoxyde de cyclohexyle (HPOCH) peut tout d'abord être réalisée par catalyse homogène, c'est-à-dire en présence d'un catalyseur dissous dans le milieu réactionnel. Ainsi le brevet FR-A-1 580 206 décrit l'oxydation d'un cycloalcane en phase liquide suivie du chauffage de la solution de l'hydroperoxyde de cycloalkyle dans le cycloalcane ainsi obtenue, en présence d'un dérivé soluble du chrome comme catalyseur. De même l'article du Journal of Molecular Catalysis (1988), 48, pages 129 à 148, décrit l'utilisation de sels organiques, tels que l'octanoate de cobalt ou de complexes, dissous dans la phase liquide organique où se déroule la réaction.

Toutefois, l'utilisation de tels sels peu coûteux présente des inconvénient car ces catalyseurs se désactivent très vite provoquant leur précipitation dans le milieu.

Pour remédier à ces problèmes, des systèmes catalytiques plus complexes ont été préconisés, tels que des complexes entre un métal et des porphyrines ou phtalocyanines. De tels systèmes sont, par exemple, décrits dans le brevet US 5 672 778, et les articles parus dans "Catalysis Letters" 20, 1993, 359-364 ou 36, 1996,69-73. Egalement des complexes à base de cobalt et de ligands analogues ont également été décrits dans l'article paru dans Journal of the American Chemical Society (1985), 107, pages 3534 à 3540

Il a également été proposé, notamment dans le brevet européen 270 468 l'utilisation de ruthénium et d'un coordinat à base de bis(pyridyl-2 imino)-1,3 isoindoline.

Ces systèmes catalytiques présentent des inconvénients liés notamment à leur stabilité (résistance à l'oxydation), leur complexité les rendant peu économiques.

Il a également été proposé de réaliser la décomposition de l'hydroperoxyde par catalyse hétérogène, c'est-à-dire en présence d'un catalyseur non dissous dans le milieu réactionnel, notamment en utilisant comme support de la phase catalytique des zéolithes ou oxydes métalliques tels que décrits dans les brevets DE19849593, FR2744719, us2851496, wo 01/44153 et dans la publication du Journal of Molec. Catal pages 15-163 du vol.79 , n°1-3 (1993). Toutefois, ces catalyseurs ont souvent une activité diminuant rapidement, cette perte d'activité étant due, par exemple, à l'élution de la phase catalytique.

Par ailleurs, il a également été proposé des catalyseurs permettant, simultanément à la décomposition de l'hydroperoxyde en alcool et/ou cétone, de réaliser une oxydation de l'hydrocarbure utilisé comme solvant permettant ainsi d'améliorer le rendement total de transformation de l'hydrocarbure en alcool et cétone. Cette oxydation est appelée dans le présent domaine technique "un transfert oxydant". Elle consiste à transférer un des atomes d'oxygène de l'hydroperoxyde sur l'hydrocarbure pour obtenir l'alcool correspondant. Le brevet européen 0331590 décrit l'utilisation d'un catalyseur homogène à base de complexes d'osmium qui permet d'obtenir de l'ordre de 40 % de transfert oxydant.

La présente invention a pour but de proposer un nouveau système catalytique hétérogène économiquement intéressant, et permettant notamment d'obtenir un niveau élevé de transfert oxydant.

Plus précisément, elle propose un procédé de décomposition d'hydroperoxydes organiques en présence d'un catalyseur en un mélange d'alcools et de cétones dans lequel le catalyseur comprend au moins un élément métallique catalytiquement actif à base de ruthénium incorporé dans un support solide choisi dans le groupe comprenant les noirs d'acétylène.

Le catalyseur de l'invention est un catalyseur hétérogène pouvant être obtenu par toutes les techniques habituelles de fabrication des catalyseurs dit supportés. Ainsi, le terme incorporé utilisé ci-dessus recouvre toutes les formes de lien entre le support et le ou les composés ou complexes de ruthénium. Ainsi, ce terme recouvre aussi bien une absorption des composés du ruthénium sur un support qu'une coprécipitation du ruthénium et d'un précurseur du support. Le ruthénium peut être simplement déposé sur la surface du support, notamment la surface d'au moins certains pores du support, ou au contraire lié audit support par des liaisons électroniques.

A titre de supports convenables pour l'invention, on peut citer les noirs d'acétylène.

Le catalyseur de l'invention peut également comprendre d'autres éléments métalliques permettant d'améliorer ou doper l'activité catalytique du ruthénium. Comme éléments métalliques convenables comme dopant, on peut citer les métaux de transition tels que ceux appartenant aux groupes 1b, 2b, 3b, 4b, 5b, 6b, 7b, 8 de la Classification Périodique des éléments telle que publiée dans Handbook of Chemistry and Physics, 66ème édition (1985/1986), de The Chemical Rubber Co.

On peut citer plus particulièrement le titane, le zirconium, le vanadium, le niobium, le tantale, le chrome, le molybdène, le tungstène, le manganèse, le rhénium, le fer, le cobalt, le nickel, le rhodium, le palladium, le platine, l'iridium, l'osmium, le cuivre, l'argent, l'or et les métaux des terres rares comme le lanthane et le cérium.

La réaction de décomposition de l'invention est conduite en phase liquide, la concentration de l'hydroperoxyde étant comprise entre 0,1 et 80 % en poids dans le milieu réactionnel. Cette concentration est avantageusement comprise entre 0, 5 et 20 %.

On peut faire appel à divers solvants tels que les alcanes parmi lesquels on citera plus particulièrement l'hexane, l'heptane et l'isooctane ; les cycloalcanes parmi lesquels on mentionnera à titre illustratif le cyclohexane et le cyclooctane, les hydrocarbures aromatiques tels le benzène, le toluène et le xylène, les hydrocarbures halogénés, et les mélanges de ces solvants

Toutefois il est à noter que l'hydroperoxyde étant généralement produit sous la forme d'une solution dans un hydrocarbure, par exemple le cyclohexane, par oxydation de celui-ci, la réaction de décomposition est avantageusement réalisée sur une solution provenant de l'oxydation de l'hydrocarbure (cyclohexane) dans laquelle la concentration en hydroperoxyde est comprise dans les limites indiquées précédemment. Cette solution peut être utilisée en l'état ou après élimination de certains constituants de manière en soi connue. Il est également possible d'utiliser une solution d'hydroperoxyde dans le solvant, par exemple, le cyclohexane sensiblement pur.

La quantité de catalyseur engagée peut être extrêmement variable, notamment en fonction des conditions de réalisation du procédé : procédé en marche continue, discontinue ou semi-continue. De manière générale, la quantité de catalyseur exprimée en pourcentage molaire de métal actif par rapport à l'hydroperoxyde à décomposer représente de 0,0001 % à 20 % et de préférence de 0,01 % à 10 %.

Dans le cadre d'une marche en continu du procédé, il est bien évident que la quantité de catalyseur par rapport à l'hydroperoxyde n'a pas de signification et que les rapports indiqués précédemment peuvent alors être beaucoup plus importants.
La température est généralement comprise entre 20 et 200°C et, de préférence, entre 80 et 130°C.

La pression atmosphérique ou supérieure à la pression atmosphérique sera suffisante pour maintenir le cyclohexane en phase liquide.

La durée de réaction (ou le temps de séjour) est généralement comprise entre quelques minutes et 4 heures et peut être ajustée compte tenu des objectifs de production, de la quantité des divers constituants du système catalytique engagée et des autres paramètres de la réaction.

En fin de réaction les produits peuvent être récupérés et/ou séparés par tout moyen approprié, par exemple par distillation.

Les hydroperoxydes qui sont mis en oeuvre dans le procédé de l'invention sont de manière générale les hydroperoxydes primaires ou secondaires dérivant des alcanes, des cycloalcanes, des hydrocarbures alkyl-aromatiques dont le cycle aromatique comporte éventuellement un ou plusieurs substituants tels que notamment groupe alkyle ou atome d'halogène plus particulièrement atome de chlore, des alcènes et des cycloalcènes ayant de 3 à 20 atomes de carbone.

A titre d'exemples de tels hydroperoxydes, on peut citer l'hydroperoxyde de cyclohexyle, l'hydroperoxyde de cyclododécyle, l'hydroperoxyde de la tétraline, l'hydroperoxyde d'éthylbenzène, l'hydroperoxyde du pinane.

Parmi ces hydroperoxydes, l'un des plus intéressants est très certainement l'hydroperoxyde de cyclohexyle dont l'oxydation conduit au cyclohexanol et à la cyclohexanone, intermédiaires dans la préparation de l'acide adipique, l'un des composés de base du polyamide 6-6.

Les exemples ci-dessous donnés uniquement à titre indicatif illustreront l'invention et ses avantages et détails.

### Exemple 1 comparatif: Synthèse catalyseur A (Ru/ZrO2 - 5% Ru massique)

Le support ZrO₂ est préalablement calciné pendant 2 heures à 500°C. Après calcination, la zircone (10 g) est placée dans 500 mL d'eau et laissée sous agitation 5 minutes à température ambiante. Le pH de la solution est ajusté à pH 9 par ajout de Na₂CO₃. On effectue ensuite une montée en température sous agitation. Une solution dans l'eau de RuCl₃,nH₂O, commercialisé par la société "STREM" (environ 20 mL pour 1.79 g de RuCl₃,nH₂O) est alors ajoutée à 90°C en 20 minutes. Le mélange est laissé sous agitation pendant 15 minutes. Le pH est de nouveau ajusté à 8/9 par addition de Na₂CO₃ et la solution est agitée pendant 3 heures à 90°C.
Le mélange est ensuite refroidi à 40-50°C puis filtré. Le gâteau est soumis à des opérations de filtration-lavage avec de l'eau jusqu'à obtention d'un pH neutre des eaux de lavage. Le solide est séché pendant 18 heures à 120°C, calciné à 400°C pendant 2 heures.

### Exemple 2 : Synthèse du catalyseur B (Ru/ noir d'acétylène Y200- 5% Ru massique)

Le support Y200 (support de noir d'acétylène commercialisé par la société SN2A) est préalablement calciné pendant 1 heure à 500°C. Après calcination, le noir d'acétylène (10 g) est placé dans 500 mL d'eau et laissé sous agitation 5 minutes à température ambiante. Le pH de la solution est ajusté à pH 9 par ajout de Na₂CO₃. On effectue ensuite une montée en température sous agitation. Une solution de RuCl₃ dans l'eau (environ 20 mL pour 1.79 g de RuCl₃) est alors ajoutée à 90°C en 20 minutes. Le mélange est laissé sous agitation pendant 15 minutes. Le pH est de nouveau ajusté à 8/9 par addition de Na₂CO₃ et la solution est agitée pendant 3 heures à 90°C.
Le mélange est ensuite refroidi à 40-50°C puis filtré. Le gâteau est soumis à des opérations de filtration-lavage avec de l'eau jusqu'à obtention d'un pH neutre des eaux de lavage. Le solide est séché pendant 18 heures à 120°C, calciné à 400°C pendant 2 heures.

### Exemple 3 : Synthèse du catalyseur C (Ru/ noir d'acétylène Y200 - 5% Ru massique)

Le support Y200 est préalablement calciné pendant 1 heure à 500°C sous air. Après calcination, le noir d'acétylène (10 g) est placé dans 300 mL de THF et laissé sous agitation 30 minutes à température ambiante. Une solution de Ru₃(CO)₁₂ dans le tétrahydrofuranne (THF) (environ 100 mL pour 1.12 g de Ru₃(CO)₁₂) est alors ajoutée à l'ambiante en 30 minutes. Le mélange est laissé sous agitation pendant 30 minutes. La solution est ensuite placée dans un évaporateur rotatif pendant 4 heures (température ambiante, pression atmosphérique, 200 tours/minute). Le THF est ensuite évaporé sous 20 mmHg. On obtient 11 g de solide.

### Exemple 4 comparatif: Synthèse du catalyseur D (Ru/ alumine- 5% Ru massique)

Le support alumine commercialisée par la société Condéa est préalablement calciné pendant 6 heures à 750°C. Après calcination, l'alumine (10 g) est placée dans 500 mL d'eau et laissée sous agitation 5 minutes à température ambiante. Le pH de la solution est ajusté à pH 9 par ajout de Na₂CO₃. On effectue ensuite une montée en température (90°C en 45 minutes) sous agitation. Une solution de RuCl₃ dans l'eau (environ 20 mL pour 1.98 g de RuCl₃) est alors ajoutée à 90°C en 20 minutes. Le mélange est laissé sous agitation pendant 15 minutes. Le pH est de nouveau remonté à 8/9 par addition de Na₂CO₃ et la solution est agitée pendant 3 heures à 90°C.
Le mélange est ensuite refroidi à 40-50°C puis filtré sur verre fritté n°4. Le gâteau est repris dans 100 mL d'eau à 40-50°C. Suivent alors 4 étapes de filtration-lavage jusqu'à obtention d'un pH neutre des eaux de lavage. Le solide est séché pendant 24 heures à 120°C sans calcination.

### Exemple 5 comparatif : Synthèse du catalyseur E (Ru/ La₂O₃ - 5% Ru massique)

Le support oxyde de lanthane (commercialisé par Rhodia) subit en premier lieu un prétraitement. Ce dernier consiste à placer l'oxyde de lanthane dans l'eau (90 mL) pendant 30 minutes avec une montée en température (90°C sur 3 heures). La solution est ensuite refroidie à 45°C, filtrée, séchée 18 heures à 120 °C puis calcinée à 400°C pendant 24 h sous air (5°C/min). Après calcination, l'oxyde de lanthane (10 g) est placé dans 500 mL d'eau et laissé sous agitation 5 minutes à température ambiante. Le pH de la solution est ajusté à pH 9 par ajout de Na₂CO₃. On effectue ensuite une montée en température (90°C en 45 minutes) sous agitation. Une solution de RuCl₃ dans l'eau (environ 20 mL pour 1.08 g de RuCl₃) est alors ajoutée à 90°C en 20 minutes. Le mélange est laissé sous agitation pendant 15 minutes. Le pH est de nouveau remonté à 8/9 par addition de Na₂CO₃ et la solution est agitée pendant 3 heures à 90°C.
Le mélange est ensuite refroidi à 40-50°C puis filtré sur verre fritté n°4. Le gâteau est repris dans 100 mL d'eau à 40-50°C. Suivent alors 4 étapes de filtration-lavage jusqu'à obtention d'un pH neutre des eaux de lavage. Le solide est séché pendant 16 heures à 120°C sans calcination.

### Exemple 7 comparatif : Synthèse du catalyseur F (Ru/ magnésie - 5% Ru massique)

Le support magnésie (UBE Industrie 100 Å) (10 g) subit en premier lieu un prétraitement. Ce dernier consiste à placer la magnésie dans l'eau (100 mL) pendant 30 minutes avec une montée en température (90°C sur 3 heures). La solution est ensuite refroidie à 45°C, filtrée, séchée 16 heures à 120 °C en étuve, puis calcinée à 400°C pendant 24 h sous air (5°C/min). La magnésie pré-traitée est placée dans 400 mL d'eau et laissée sous agitation 5 minutes à température ambiante. Le pH de la solution est ajusté à pH 9 par ajout de Na₂CO₃. On effectue ensuite une montée en température (90°C en 45 minutes) sous agitation. Une solution de RuCl₃ dans l'eau (environ 20 mL pour 1.08 g de RuCl3) est alors ajoutée à 90°C en 20 minutes. Le mélange est laissé sous agitation pendant 15 minutes. Le pH est de nouveau remonté à 8/9 par addition de Na₂CO₃ et la solution est agitée pendant 3 heures à 90°C.
Le mélange est ensuite refroidi à 40-50°C puis filtré sur verre fritté n°4. Le gâteau est repris dans 100 mL d'eau à 40-50°C. Suivent alors 4 étapes de filtration-lavage jusqu'à obtention d'un pH neutre des eaux de lavage. Le solide est séché pendant 16 heures à 120°C sans calcination.

### Exemple 8 comparatif : Synthèse du catalyseur G (Ru/ magnésie - 5% Ru massique)

Le support magnésie (UBE Industrie 100 Å) (15 g) subit en premier lieu un prétraitement. Ce dernier consiste à placer la magnésie dans l'eau (150 mL) pendant 30 minutes avec une montée en température jusqu'à 90°C sur 3 heures. La solution est ensuite refroidie à 45°C, filtrée, séchée 16 heures à 120 °C en étuve, puis calcinée à 400°C pendant 24 h sous air (5°C/min). La magnésie pré-traitée est placée dans 150 mL d'eau et laissée sous agitation 10 minutes à température ambiante. Une solution de RuCl₃ dans l'eau (environ 20 mL pour 1.62 g de RuCl₃) est alors ajoutée à température ambiante en 15 minutes. Le mélange est laissé sous agitation pendant 1 h30 à température ambiante. Après une décantation, l'eau est ensuite éliminée à l'évaporateur rotatif sous 20 mmHg à 50°C. Le gâteau est séché à l'étuve pendant 16 heures à 120°C. On ajoute alors 41 mL de NaOH 0.1 M au gâteau afin que celui-ci puisse être agité. La solution obtenue est alors chauffée à 95°C pendant 3 heures. On ajoute 50 mL de soude 0.1 M et on laisse refroidir la solution à 40-50°C. Après deux lavages de 100 mL d'eau à 45°C, le solide est séché pendant 16h à 120°C à l'étuve. Le solide est enfin repris dans 400 mL d'eau à température ambiante, agité pendant 30 minutes, chauffé à 70°C pendant 3 heures, puis centrifugé (pH phase aqueuse = 7) à 3500 tours/minutes pendant 20 minutes. Le solide ainsi obtenu est séché 16 heures à 120°C.

### Exemple 9 : Synthèse du catalyseur H (Rut noir d'acétylène Y70 (SN2A) - 5% Ru massique)

Le support noir d'acétylène Y70 SN2A (10 g) est tout d'abord calciné 1 heure à 500°C. Le noir (10 g) est placé dans 400 mL d'eau et laissé sous agitation 5 minutes à température ambiante. Le pH de la solution est ajusté à pH 9 par ajout de Na₂CO₃. On effectue ensuite une montée en température (90°C en 45 minutes) sous agitation. Une solution de RuCl₃ dans l'eau (environ 20 mL pour 1.08 g de RuCl₃) est alors ajoutée à 90°C en 20 minutes. Le mélange est laissé sous agitation pendant 15 minutes. Le pH est de nouveau remonté à 8/9 par addition de Na₂CO₃ et la solution est agitée pendant 3 heures à 90°C.
Le mélange est ensuite refroidi à 40-50°C puis filtré sur verre fritté n°4. Le gâteau est repris dans 100 mL d'eau à 40-50°C. Suivent alors 4 étapes de filtration-lavage jusqu'à obtention d'un pH neutre des eaux de lavage. Le solide est séché pendant 16 heures à 120°C sans calcination.

### Exemple 10 comparatif : Synthèse du catalyseur J (Ru/ Cérine (HSA5) - 5% Ru massique)

Le support cérine HSA5 (commercialisé par Rhodia) (10.2 g) est tout d'abord calciné 6 heures à 500°C sous air. L'oxyde de cérium (10.2 g) est placé dans 100 mL d'eau et une solution de Ru(acac)₃ anhydre dans l'acétone (environ 100 mL pour 2 g de Ru(acétylacétonate)₃) est alors ajoutée à l'ambiante. Le mélange est laissé sous agitation pendant 2 heures à l'ambiante. La solution est concentrée à l'évaporateur rotatif à 45°C, puis séchée 16 heures à l'étuve à 120°C. Sur la gâteau sec, on ajoute alors 41 mL de soude 0.1 M et on chauffe sous agitation pendant 3 heures à 95°C. Une fois refroidi à 40-50°C, le produit est filtré, puis lavé 4 fois à 45°C par 100 mL d'eau. Le solide est séché 16 heures à 120°C à l'étuve. Le solide est ensuite repris dans 210 mL d'eau à l'ambiante puis chauffé 3 heures à 70°C. Après refroidissement à 45°C, le solide est filtré puis séché 16 heures à 120°C.

### Exemple 11 : Synthèse du catalyseur K (Ru dopé Fe sur noir d'acétylène Y200)

Le support Y200 SN2A (10 g) est calciné 1 heure à 500°C. Le noir d'acétylène (10 g) est placé dans 400 mL d'eau et agité 5 minutes. Le mélange est porté progressivement à 90°C (en 45 minutes) et une solution de FeCl₃, 6 H₂O (0.509 g dans 50 mL d'eau) est ajouté sut le noir d'acétylène à 90°C en 13 minutes. Le pH de la solution est ramené à 5.5 par addition de bicarbonate de sodium. La solution est maintenue 30 minutes sous agitation à 90°C. Après refroidissement à 45°C, on effectue 4 lavages successifs avec 100 mL d'eau à 45°C. Le produit est séché 16 heures à 120°C en étuve. Le solide est remis dans le réacteur en présence de 400 mL d'eau, est chauffé à 90°C et on ajuste le pH à 10 avec Na₂CO₃. On maintient l'agitation 1 heure à 90°C. Une solution de RuCl₃ ALPHA dans l'eau (environ 20 mL pour 1.08 g de RuCl₃) est alors ajoutée en 20 minutes à 90°C. Le mélange est laissé sous agitation pendant 3 heures à 90°C. Une fois refroidi à 40-50°C, le produit est filtré, puis lavé .et filtré 4 fois successives afin d'obtenir un pH des eaux de lavage neutre. Le solide est séché 16 heures à 120°C à l'étuve.

### Exemple 12 : Synthèse du catalyseur L (Ru dopé Co sur noir d'acétylène Y200)

Le support Y200 SN2A (10 g) est calciné 1 heure à 500°C. Le noir d'acétylène (10g) est placé dans 400 mL d'eau et agité 5 minutes. Le mélange est porté progressivement à 90°C (en 45 minutes) et on ajoute du bicarbonate de soude afin d'atteindre pH 10. Une solution de RuCl₃ ALPHA dans l'eau (environ 20 mL pour 1.08 g de RuCl₃) est alors ajoutée en 20 minutes à 90°C. Le mélange est laissé sous agitation pendant 1 heures à 90°C. Une solution de CoCl₂, 6 H₂O (1.429 g dans 20 mL d'eau) est ensuite ajoutée dans le réacteur à 90°C. La solution est maintenue 1 heure sous agitation à 90°C. Après refroidissement à 45°C, le solide est filtré sur verre fritté n°4 . Le gâteau est repris dans 100 mL d'eau à 40-50°C et on effectue 4 lavages successifs afin d'obtenir un pH des eaux de lavage neutre. Le solide est séché 16 heures à 120°C à l'étuve.

### Exemples 13 à 24 : Résultats catalytiques en déperoxydation de l'HPOCH

Les catalyseurs supportés ont été testés dans les conditions classiques de déperoxydation : 40 g d'oxydat provenant de l'auto-oxydation du cyclohexane (5% d'HPOCH, comme décrit dans le brevet FR2087375) sont mis en présence d'environ 170 mg de catalyseur hétérogène pendant plusieurs heures à 80°C dans un réacteur surmonté d'un Dean Stark (élimination en continu de l'eau formée dans le milieu réactionnel). Les catalyseurs (5% massique en Ru) ont été utilisés avec des rapports molaires Ru/HPOCH = 0.5%. Les dosages d'HPOCH résiduel sont effectués par dosage potentiométrique en retour (Iode /thiosulfate) et les dosages de cyclohexanol (Ol) et de cyclohexanone (One) par CPG. Les résultats sont consignés dans le tableau ci-après. On entend par TT (taux de transformation), le rapport du nombre de moles d'HPOCH converti sur le nombre de mole d'HPOCH initial.

| Exemple | Cata n° | Métal | Support | t (min) | TT (%) | One/Ol |
|---|---|---|---|---|---|---|
| Exemple 13 | A | Ru | Zircone | 60 | 97.4 | / |
| Exemple 14 | B | Ru | Noir d'acétylène Y200 | 50 | 97.7 | 0.52 |
| Exemple 15 | C | Ru | Noir d'acétylène Y200 | 60 | 97.3 | 0.55 |
| Exemple 16 | D | Ru | Alumine | 90 | 97.1 | 0.52 |
| Exemple 17 | E | Ru | Oxyde de lanthane | 60 | 97.7 | 0.52 |
| Exemple 19 | F | Ru | Magnésie | 270 | 97.8 | 0.53 |
| Exemple 20 | G | Ru | Magnésie | 300 | 95.3 | 0.53 |
| Exemple 21 | H | Ru | Noir d'acétylène Y70 | 110 | 93.5 | 0.48 |
| Exemple 22 | J | Ru | Cérine | 45 | 97.9 | 0.63 |
| Exemple 23 | K | Ru/Fe | Noir d'acétylène Y200 | 90 | 95,8 | 0,48 |
| Exemple 24 | L | Ru/Co | Noir d'acétylène Y200 | 120 | 94,5 | 0,47 |

## Revendications

1. Procédé de décomposition d'hydroperoxydes organiques en présence d'un catalyseur en alcools et cétones, **caractérisé en ce que** le catalyseur comprend comme élément métallique catalytiquement actif du ruthénium incorporé sur un support choisi dans le groupe comprenant les noirs d'acétylène.

2. Procédé selon la revendication 1, **caractérisé en ce que** le catalyseur comprend au moins un élément métallique supplémentaire comme élément dopant.

3. Procédé selon la revendication 2, **caractérisé en ce que** l'élément métallique dopant est choisi dans le groupe comprenant les métaux de transition des groupes 1b, 2b, 3b, 4b, 5b, 6b, 7b, 8 de la Classification Périodique des éléments telle que publiée dans Handbook of Chemistry and Physics, 66^{ème} édition (1985/1986), de The Chemical Rubber Co.

4. Procédé selon la revendication 3, **caractérisé en ce que** l'élément métallique dopant est choisi dans le groupe comprenant les métaux des terres rares, le titane, le zirconium, le vanadium, le niobium, le tantale, le chrome, le molybdène, le tungstène, le manganèse, le rhénium, le fer, le cobalt, le nickel, le rhodium, le palladium, le platine, l'or, l'argent, le cuivre, l'iridium, l'osmium.

5. Procédé selon l'une des revendications précédentes, **caractérisé en ce que** la quantité de catalyseur exprimée en pourcentage molaire de ruthénium par rapport au nombre de mole d'hydroperoxyde à décomposer représente de 0,0001% à 20%.

6. Procédé selon la revendication 1 à 5, **caractérisé en ce que** la température à laquelle est réalisée la réaction de décomposition est comprise entre 20°C et 200°C.

7. Procédé selon l'une des revendications 1 à 6, **caractérisé en que** la réaction est conduite dans un solvant de l'hydroperoxyde.

8. Procédé selon la revendication 7, **caractérisé en que** le solvant est un hydrocarbure, un alcane, un hydrocarbure halogéné ou un mélange de ces solvants.

9. Procédé selon la revendication 8, **caractérisé en que** le solvant est un hydrocarbure identique à celui dont l'oxydation conduit à l'hydroperoxyde à décomposer.

10. Procédé selon l'une des revendications 7 à 9, **caractérisé en que** la concentration de l'hydroperoxyde se situe entre 1 % et 80 % en poids par poids de solution.

11. Procédé selon l'une des revendications 1 à 10, **caractérisé en que** l'hydroperoxyde mis en oeuvre est choisi parmi les hydroperoxydes primaires ou secondaires dérivant des alcanes, des cycloalcanes, des hydrocarbures alkyl-aromatiques, des alcènes et des cycloalcènes ayant de 3 à 20 atomes de carbone.

12. Procédé selon la revendication 11, **caractérisé en que** les hydrocarbures alkyl-aromatiques comporte un ou plusieurs substituants choisis dans le groupe comprenant les groupes alkyles ou les atomes d'halogènes

13. Procédé selon l'une des revendications 1 à 12, **caractérisé en que** l'hydroperoxyde est choisi dans le groupe comprenant l'hydroperoxyde de cyclohexyle, l'hydroperoxyde de cyclododécyle, l'hydroperoxyde de la tétraline, l'hydroperoxyde d'éthylbenzène, l'hydroperoxyde du pinane.

## Claims

1. Process for decomposing organic hydroperoxides in the presence of a catalyst into alcohols and ketones, **characterized in that** the catalyst comprises, as catalytically active metallic element, ruthenium incorporated onto a support chosen from the group comprising acetylene blacks.

2. Process according to Claim 1, **characterized in that** the catalyst comprises at least one additional metallic element as dopant element.

3. Process according to Claim 2, **characterized in that** the dopant metallic element is chosen from the group comprising the transition metals of Groups 1b, 2b, 3b, 4b, 5b, 6b, 7b and 8 of the Periodic Table of Elements as published in *Handbook of Chemistry and Physics,* 66^{th} edition (1985/1986) by The Chemical Rubber Co.

4. Process according to Claim 3, **characterized in that** the dopant metallic element is chosen from the group comprising the rare-earth metals, titanium, zirconium, vanadium, niobium, tantalum, chromium, molybdenum, tungsten, manganese, rhenium, iron, cobalt, nickel, rhodium, palladium, platinum, gold, silver, copper, iridium and osmium.

5. Process according to one of the preceding claims, **characterized in that** the amount of catalyst expressed as a molar percentage of ruthenium with respect to the number of moles of hydroperoxide to be decomposed represents from 0.0001% to 20%.

6. Process according to one of Claims 1 to 5,
**characterized in that** the temperature at which the decomposition reaction is carried out is between 20°C and 200°C.

7. Process according to one of Claims 1 to 6,
**characterized in that** the reaction is carried out in a solvent for the hydroperoxide.

8. Process according to Claim 7, **characterized in that** the solvent is a hydrocarbon, an alkane, a halogenated hydrocarbon or a mixture of these solvents.

9. Process according to Claim 8, **characterized in that** the solvent is a hydrocarbon identical to that whose oxidation leads to the hydroperoxide to be decomposed.

10. Process according to one of Claims 7 to 9, **characterized in that** the hydroperoxide concentration lies between 1% and 80% by weight with respect to the weight of solution.

11. Process according to one of Claims 1 to 10,
**characterized in that** the hydroperoxide used is chosen from primary or secondary hydroperoxides derived from alkanes, cycloalkanes, aromatic alkyl hydrocarbons, alkenes and cycloalkenes having from 3 to 20 carbon atoms.

12. Process according to Claim 11, **characterized in that** the aromatic alkyl hydrocarbons have one or more substituents chosen from the group comprising alkyl groups or halogen atoms.

13. Process according to one of Claims 1 to 12,
**characterized in that** the hydroperoxide is chosen from the group comprising cyclohexyl hydroperoxide, cyclododecyl hydroperoxide, tetralin hydroperoxide, ethylbenzene hydroperoxide and pinane hydroperoxide.

## Patentansprüche

1. Verfahren zur Zersetzung von organischen Hydroperoxiden in Gegenwart eines Katalysators zu Alkoholen und Ketonen, **dadurch gekennzeichnet, daß** der Katalysator als katalytisches wirksames Metallelement auf einem Träger aus der Gruppe bestehend aus Acetylenrußen eingearbeitetes Ruthenium enthält.

2. Verfahren nach Anspruch 1, **dadurch gekennzeichnet, daß** der Katalysator mindestens ein zusätzliches Metallelement als Dotierelement enthält.

3. Verfahren nach Anspruch 2, **dadurch gekennzeichnet, daß** man das Dotiermetallelement aus der Gruppe bestehend aus Übergangsmetallen der Gruppen 1b, 2b, 3b, 4b, 5b, 6b, 7b und 8 des Periodensystems der Elemente gemäß Handbook of Chemistry and Physics, 66. Auflage (1985/1986), von The Chemical Rubber Co. auswählt.

4. Verfahren nach Anspruch 3, **dadurch gekennzeichnet, daß** man das Dotiermetallelement aus der Gruppe bestehend aus Seltenerdmetallen, Titan, Zirconium, Vanadium, Niob, Tantal, Chrom, Molybdän, Wolfram, Mangan, Rhenium, Eisen, Cobalt, Nickel, Rhodium, Palladium, Platin, Gold, Silber, Kupfer, Iridium und Osmium auswählt.

5. Verfahren nach einem der vorhergehenden Ansprüche, **dadurch gekennzeichnet, daß** die Katalysatormenge, ausgedrückt in Molprozent Ruthenium in bezug auf die Zahl der Mole des zu zersetzenden Hydroperoxids 0,0001% bis 20% beträgt.

6. Verfahren nach Anspruch 1 bis 5, **dadurch gekennzeichnet, daß** die Temperatur, bei der die Zersetzungsreaktion durchgeführt wird, zwischen 20°C und 200°C liegt.

7. Verfahren nach einem der Ansprüche 1 bis 6,
**dadurch gekennzeichnet, daß** die Reaktion in einem Lösungsmittel für das Hydroperoxid durchgeführt wird.

8. Verfahren nach Anspruch 7, **dadurch gekennzeichnet, daß** es sich bei dem Lösungsmittel um einen Kohlenwasserstoff, ein Alkan, einen halogenierten Kohlenwasserstoff oder eine Mischung dieser Lösungsmittel handelt.

9. Verfahren nach Anspruch 8, **dadurch gekennzeichnet, daß** es sich bei dem Lösungsmittel um einen Kohlenwasserstoff handelt, der mit demjenigen, dessen Oxidation zu dem zu zersetzenden Hydroperoxid führt, identisch ist.

10. Verfahren nach einem der Ansprüche 7 bis 9,
**dadurch gekennzeichnet, daß** die Hydroperoxidkonzentration zwischen 1 Gew.-% und 80 Gew.-%, bezogen auf das Gewicht der Lösung, liegt.

11. Verfahren nach einem der Ansprüche 1 bis 10, **dadurch gekennzeichnet, daß** man das verwendete Hydroperoxid unter primären oder sekundären Hydroperoxiden, die sich von Alkanen, Cycloalkanen, alkylaromatischen Kohlenwasserstoffen, Alkenen und Cycloalkenen mit 3 bis 20 Kohlenstoffatomen ableiten, auswählt.

12. Verfahren nach Anspruch 11, **dadurch gekennzeichnet, daß** die alkylaromatischen Kohlenwasserstoffe einen oder mehrere Substituenten aus der Gruppe bestehend aus Alkylgruppen oder Halogenatomen enthalten.

13. Verfahren nach einem der Ansprüche 1 bis 12,
**dadurch gekennzeichnet, daß** man das Hydroperoxid aus der Gruppe bestehend aus Cyclohexylhydroperoxid, Cyclododecylhydroperoxid, Tetralinhydroperoxid, .Ethylbenzolhydroperoxid und Pinanhydroperoxid auswählt.
